# EUROPEAN PATENT SPECIFICATION

(11) **EP 2 547 325 B1**
(45) Date of publication and mention of the grant of the patent: **24.06.2020**
(21) Application number: 11712041.0
(22) Date of filing: 17.03.2011
(51) Int. Cl.: A61K 9/12, A61K 47/02

(54) **SPRAY-PUMPABLE COMPOSITION SUITABLE FOR TOPICAL SKIN APPLICATION**
SPRÜHPUMPZUSAMMENSETZUNG FÜR TOPISCHE ANWENDUNG AUF DER HAUT
COMPOSITION POMPABLE POUR ATOMISEUR, APPROPRIÉE POUR UNE APPLICATION TOPIQUE SUR LA PEAU

(30) Priority: 19.03.2010 NL 2004437
(43) Date of publication of application: 23.01.2013
(73) Proprietor: Forte IQ B.V., 6584 AC Molenhoek (NL)
(72) Inventor: WILLEMS, Maria, Margaretha, Martina, NL-5432 GP Cuijk (NL); VAN DE LOCKAND, Petrus, Richardus, Marinus, NL-5434 SL Vianen (NL); MEIJLINK, Peter, NL-6584 AL Molenhoek (NL)
(74) Representative: van Someren, Petronella F. H. M.
(86) International application number: PCT/NL2011/050186
(87) International publication number: WO 2011/115489

(56) References cited:
- EP-A2- 1 837 007
- DE-A1- 2 503 962
- GB-A- 2 338 650
- A Burger, H Wachter: "Hunnius' pharmazeutisches Wörterbuch", 1 January 1993 (1993-01-01), Walter de Gruyter, Berlin, New York ISBN: 3-11-013868-9 * page 1293 *

## Description

The present disclosure relates to a spray-pumpable composition for topical use on the skin, a method for the preparation thereof and compositions for the treatment of skin disorders.

Compositions for topical use on the skin comprising considerable amounts of active ingredients, such as zinc oxide, are known in the art. In general such compositions are in the form of a rather thick mass, such as a cream or ointment. These compositions are applied to the skin by taking an amount thereof on one or more fingertips followed by firmly rubbing of the composition over the targeted skin. It will be clear that such compositions are not optimized for topical skin applications where touching of sore or otherwise adversely affected skin should be minimized to not cause any unnecessary additional discomfort.

Another point of concern is that, although such compositions may have lubricating properties, spreading thereof over the skin may not result in a sufficiently thin and/or evenly spread layer but rather results in an uneven, patch-like distribution of the composition over the skin. The consequence thereof may be that a residue of the active ingredients comprised by the applied composition is left on the skin in an undesired, non-uniform pattern and/or results in an insufficient local dosage. As such compositions may not allow even spreading thereof on the skin, a considerable amount of the composition would need to be applied at once, or may require a second or further dosage, in order to cover the targeted skin area in a sufficient manner.

Another complication may arise when circumstances require a significant amount of an active ingredient to be applied onto the affected skin area. In such cases it would be highly beneficial if a composition developed for topical use on the skin would be available which would be spray-pumpable and contain the respective active ingredient in such a high concentration that a single topical skin application or treatment would ensure that the active ingredient would be present on the skin in the desired dosage. However, addition of high amounts of certain ingredients to compositions for topical skin applications can adversely affect the suitability of the composition for topical use, in particular the properties which ensure the composition remains spray-pumpable.

Currently, there are hand pump systems available that allow compositions to be dispensed directly onto the skin. However, dispensing of such compositions results in the generation of a rather thick blob which subsequently requires spreading thereof over the skin. Consequently, considerable rubbing of the dispensable composition may still be required which may cause unnecessary additional discomfort.

Furthermore, present day compositions for topical use on the skin are known. However, several such compositions, such as the substantially anhydrous composition disclosed in US2005/0266035 A1 or the compositions disclosed in EP1837007-A2, DT2503962-A1 and GB2338650-A1 comprise nano-scale sized zinc oxide and/or titanium dioxide particles which are typically hydrophilized. The use of nanometre sized particles is controversial as they are sufficiently small to be able to penetrate or traverse the skin barrier. When such lipophilic particles enter the human body, they may accumulate in adipose tissue. Currently there are indications or at least concerns that the application of such small sized particles may have long-term negative health effects.

EP1837007-A2 discloses sprayable topical lotions against sunburn, comprising glyceryl stearate citrate, C18-36 acid triglyceride and colloidal silica coated titanium dioxide.

DE2503962-A1 discloses a topical spray pumpable formulations, comprising colloidal silica, a starch derivative, hydrocortison, citric acid ether, cremophor, ethanol and water. These compositions are able to comprise up to 5% weight percentage of active ingredient.

GB2338650-A1 discloses pump spray formulations comprising 2 to 15% of particulate solid (zinc oxide, titanium dioxide), petrolatum, polyglyceryl-3methylglucose distearate, glyceryl stearate, PEG-30 stearate and multiple parabens. Exemplified are a zinc oxide cream, a medicated nappy spray and a titanium dioxide sunscreen. The resulting O/W emulsion is spray pumpable. In this disclosure, the problem of maintaining a viscosity that allows the composition to be sprayed onto the skin is solved by preparing a thixotropic composition. This however requires the addition of Xantam gum as excipient.

Furthermore, compositions comprising (cortico)steroids as active ingredients are available, but such compositions are specifically developed for delivery of these hormones to the nasal cavity. Such compositions are not designed for use as a spray-pumpable composition for topical use on the skin.

Consequently, there remains a need to provide compositions which can be used for delivery of a wider pallet of compounds, which compositions exhibit excellent or improved properties with respect to their topical usage on the skin.

It is an object of the present invention to provide a spray-pumpable composition which comprises increased levels of one or more active ingredients, in particular zinc oxide, titanium dioxide and/or steroids, which has excellent or improved properties for topical use on the skin. It is a further object of the present invention to provide a composition which allows the application of increased amounts of active ingredients on the skin of a subject, which composition provides a means to alleviate the experience of unnecessary additional discomfort for said subject. Moreover, it is an object of the present invention to provide a method for the preparation of a composition which comprises increased levels of one or more active ingredients for topical skin use. Yet another object of the present invention is to provide spray-pumpable compositions for topical use on the skin which comprise increased levels of zinc oxide and/or titanium dioxide, which compositions are safer to use in the sense that they would not suffer from long-term adverse side-effects as a result of the inclusion of said zinc oxide and/or titanium dioxide.

This object, among others, is achieved by the composition and method according to the present invention according to the appended claims.

More in particular, this object is achieved by the provision of a spray-pumpable composition for topical use comprising unbound hydrophilic silicon dioxide particles, an emulsifier, at least one active ingredient and optionally one or more additives, wherein the at least one active ingredient comprises 5-15 weight percent zinc oxide or titanium dioxide.

An advantage of the composition and method according to the present invention is that it is now possible to provide a composition which is spray-pumpable, suitable for topical use on the skin, which is liquid under ambient conditions and which allows to keep metal oxide particles, such as zinc oxide and titanium dioxide particles, and/or other active ingredients, finely dispersed and suspended in an oil-in-water emulsion. Furthermore, the composition is sufficiently versatile and flexible to allow the addition of further additives which may increase its suitability as a spray-pumpable composition and increase its usefulness in topical skin applications. The composition according to the invention even remains of the preferred viscosity after the addition of considerable amounts of active ingredients and/or additives, without having to include a viscosity-modifying agent, even in the case when the composition comprises 5, 10 or 15 percent zinc oxide. Another advantage of the composition according to the invention is that there is a minimal requirement for shaking of these compositions prior to application on the skin.

The composition further allows obtaining a spray-pumpable composition which exhibits excellent properties with respect to its use in topical skin applications. For instance after spraying on the skin, the composition can be quickly and evenly spread over the skin to form a thin and even layer. Likely as a consequence of the low viscosity of the composition, there does not appear to remain any residue in an undesired, patch-like manner. Furthermore, the composition readily moisturizes the skin and exhibits good skin-adherence. Another advantage of the compositions of the invention is that hygienic topical application of the composition on the skin is now possible.

According to a preferred embodiment of the invention, the composition comprises hydrophilic silicon dioxide which has a specific surface area of between 130 and 380 m²/g, preferably between 150 and 300 m²/g, more preferably between 175 and 225 m²/g, most preferably around 200 m²/g.

According to a further preferred embodiment, the hydrophilic silicon dioxide is present in the composition in a concentration of 0.01-0.5 weight percent, preferably in a concentration of about 0.1 weight percent. Non-limited examples of hydrophilic silicon dioxide include Cab-o-sil, Aerosil, or Aerosil 200.

The composition according to the invention, comprising the hydrophilic silicon dioxide, is a suspension of hydrophilic silicon dioxide in an emulsion. In a preferable embodiment, the composition is a suspension of hydrophilic silicon dioxide in an oil-in-water emulsion. In a more preferable embodiment, the hydrophilic silicon dioxide is suspended in the water phase of an emulsion, in particular an oil-in-water emulsion. In an even more preferable embodiment, the hydrophilic silicon dioxide is suspended or dispersed in the water phase of an oil-in-water emulsion. In this system, the hydrophilic silicon dioxide particles according to the invention act to assure that the homogeneity of the suspension as an oil-in-water emulsion can be maintained and avoids settling of active ingredients.

The silicon dioxide is present in the composition as "unbound particles". For the purpose of this invention, this means that these particles are in essence not bound to any of the other ingredients that are included in the spray-pumpable composition. It is in particular meant that these particles are not bound to other ingredients of the composition through covalent bonds or non-covalent bonds such as ionic bonds. Furthermore, these hydrophilic silicon dioxide particles are not used as a coating to coat other ingredients of the composition, such as the zinc oxide or titanium dioxide particles. Thus, not taking into consideration the usual weak attractions which arise when hydrophilic silicon dioxide particles are contacted with water molecules, these particles are herein considered unbound and as a consequence thereof present as freely dispersed particles in the hydrophilic phase of the composition. These particles are not attached to any of the components present in the composition or associated therewith through a strong intermolecular bond.

Active ingredients can be present as a dispersion or suspension in the oil-in-water emulsion. This present invention thus allows obtaining a spray-pumpable composition for topical skin application in which active ingredients can be homogeneously suspended and stabilized.

The composition may have a water content of at least 50, 55, 60, 70, 80, or 85 weight percent. This percentage depends, among others, on the amount of active ingredients that is included in the composition.

The compositions according to the invention can be sprayed on the skin using several spray pump systems. Representative and preferred pump-spray systems which can be used, and which are herein mentioned by way of example only, have a spray volume between 0.10 ml and 0.30 ml, preferably between 0.15 and 0.25 ml, more preferably between 0.15 and 20 ml, most preferably about 0.20 ml. Preferred spray-pump systems include commercially available systems such as, but not limited to, spray-pumpable systems from Emsar®, Calmar® or Seaquest®.

Another aspect of the invention relates to the active ingredients which can be part of the composition, wherein the at least one active ingredient comprises 5-15 weight percent zinc oxide or titanium dioxide.

Other active ingredients may be selected from zinc oxide, titanium dioxide, a steroid hormone, in particular a corticosteroid hormone, an antimicrobial agent, an antibacterial agent, an antifungal agent, an antibiotic or any combination thereof.

With respect to the addition of zinc oxide, it was surprisingly found that a composition according to the invention could be obtained which comprises at least 5, preferably 10, or more preferably even 15 weight percent zinc oxide, which composition still exhibited the preferred viscosity as meant herein.

Stably suspended in the composition of the invention, are the lipophilic zinc oxide particles which were added to the lipophilic phase according to the method of the invention, providing a composition with up to at least 10 weight percent zinc oxide. Additionally or alternatively, 5 weight percent zinc oxide particles could surprisingly be dispersed in the aqueous phase of the method of the present invention which allowed the provision of a composition comprising up to 15 weight percent zinc oxide which still exhibited the viscosity as meant herein. This up to 15 weight percent zinc oxide comprising composition, or any other composition obtained via addition of zinc oxide via both phases, likely comprises zinc oxide in a unique distribution in both the lipophilic and hydrophilic phase.

However, without being bound by theory, it is believed that the dispersed or suspended hydrophilic silicon dioxide according to the invention, in combination with an emulsifier as mentioned herein, enables to obtain the elevated micron-scale sized zinc oxide concentrations of 5, 10 or even 15 weight percent in the form of a stabilized suspension in an oil-in-water emulsion. As a result of the presence of free, or unbound, hydrophilic silicon dioxide particles as meant herein, there is no requirement to change the hydrophobicity or hydrophilicity of active ingredient according to the present invention, such as zinc oxide or titanium dioxide particles. It is expected that the provision of composition having elevated levels of titanium dioxide particles is also possible.

The zinc oxide particles as meant herein encompass zinc oxide particles of size distribution which in essence fall within the range of 45 to 90 micron. More preferably, at least 99% of the particles falls within the 45-90 micron size window. Preferably, these zinc oxide particles have a specific surface area (BET) of between 4.5 and 6.9 m²/g, more preferably about 6.5 m²/g. In the dry state, these zinc oxide particles according to the invention exhibit lipophilic properties. "Zinc oxide" is used interchangeably herein with the term "zinc oxide particles".

The zinc oxide particles according to the invention can be obtained using known processes such as physical vapor synthesis, grinding or micronization.

Thus, notwithstanding the existence of compositions comprising zinc oxide particles, perhaps even in high amounts, this is believed to be the first disclosure of a spray-pumpable composition having the preferred viscosity as meant herein, without the need to include a viscosity-modifying ingredient, which is suitable for topical applications and which comprises micron-scale sized zinc oxide particles in a concentration of up to 15 weight percent. The zinc oxide composition according to the invention would not suffer from long-term adverse health effects because micron-scale sized zinc oxide particles are present in the composition instead of hydrophilized or unmodified nanoscale-sized particles. This would also apply to compositions comprising titanium dioxide particles having the same size distribution.

Whether a zinc oxide or titanium dioxide comprising composition is spray-pumpable as meant herein can be established on the basis of its viscosity. Using a straight-forward laboratory test which is based on a standardized flow viscosity meter, this test provides a means to assess whether a composition is spray-pumpable within the meaning of the invention. Using this standard flow viscosity meter, the viscosity of the composition is determined in a laboratory set-up wherein the composition under investigation is allowed to pass, as a consequence of the earths gravitational field, through a nozzle DIN 4 which has a 4 mm outflow opening.

Thus, a zinc oxide or titanium comprising composition is spray-pumpable as defined herein when a volume of 100 ml of the composition takes preferably between 10 and 30 seconds, more preferably between 12-24 seconds, most preferably between 14 and 20 seconds to flow through the DIN 4 nozzle of the standardized flow viscosity meter under ambient conditions.

Compositions which take longer to flow through the nozzle under these conditions are more difficult to be sprayed, or may not be sprayable anymore.

The composition may be an aqueous liquid having water as a main component. Herein described is a composition which is an oil-in-water emulsion comprising zinc oxide and/or titanium dioxide and has a water content of at least 50 percent by weight, preferably at least 55, more preferably at least 60 weight percent, most preferably around 65 weight percent or around 70 weight percent.

In another preferred embodiment, the composition comprises an emulsifier which is a hydrophilic or lipophilic non-ionic surfactant, preferably a lipophilic non-ionic surfactant, more preferably a gluco-lipid emulsifier or a gluco-lipid co-emulsifier and wherein the emulsifier preferably comprises cetearylalcohols and/or cetearylglucosides or C₁₄-C₂₂ alcohols and/or C₁₂-C₂₀ glucosides or a mixture thereof and is preferably present in a concentration of 0.1-10 weight percent, preferably 0.5-5 weight percent, more preferably 1-4 weight percent and most preferably about 3 weight percent.

Using an emulsifier which comprises C₁₄-C₂₂ alcohols and/or C₁₂-C₂₀ glucosides, preferably as a main component, it was found that a spray-pumpable composition can be obtained which comprises at least 5, 10 or 15 weight percent zinc oxide, which composition remains of the preferred viscosity as defined herein. These highly concentrated zinc oxide compositions of preferred viscosity also exhibit the preferred properties as meant herein which render the composition particularly suitable for topical use on the skin.

Additionally to the use of zinc oxide and/or titanium dioxide, the hydrophilic silicon dioxide comprising composition of the present invention may comprise active ingredients selected from a steroid hormone, in particular a corticosteroid hormone, an antimicrobial agent, an antibacterial agent, an antifungal agent, an antibiotic or any combination thereof.

Also these (cortico)steroids, antimicrobial agents, antibacterial agents and antifungal agents can be taken-up in the composition for topical use on the skin as claimed. Furthermore, this is believed to be the first composition which exhibits the viscosity as meant herein which is specifically designed for topical application and thus allows topical application of (cortico)steroids, antimicrobial agents, antibacterial agents and antifungal agents. Furthermore, prior to application of the composition on the skin, there is no need for vigorous, prolonged shaking of the composition. Only mild shaking of the composition prior to application suffices.

Where the composition according to the invention includes a steroid hormone, in particular a corticosteroid hormone, an antimicrobial agent, an antibacterial agent, an antifungal agent, an antibiotic or any combination thereof, is an oil-in-water emulsion having a water content of at least 60 percent by weight, preferably at least 70, more preferably at least 80 weight percent, most preferably around 85 weight percent.

The steroid hormone which can be comprised by the composition is selected from the group consisting of hydrocortisone, hydrocortisone acetate, hydrocortisone valerate, hydrocortisone butyrate, cortisone acetate, tixocortol pivalate, prednisolone, methyprednisolone, methylprednisolone aceponate, prednisone, triamcinolone acetonide, triamcinolone alcohol, amcinonide, budesonide, desonide, fluocinonide, fluocinolone acetonide, halcinonide, betamethasone, betamethasone sodium phosphate, desoximetasone, dexamethasone, dexamethasone sodium phosphate, fluocortolone, hydrocortisone-17-butyrate, hydrocortisone-17-valerate, aclometasone dipropionate, betamethasone valerate, betamethasone dipropionate, prednicarbate, clobetasone butyrate, clobetasone-17-butyrate, clobetasol-17-propionate, clobetasol diproprionate, clobetasol propionate, fluocortolone caproate, fluocortolone pivalate, halbetasol proprionate, clobetasol diproprionate, diflorasone diacetate, mometasone furoate, fluticasone proprionate, flurandrenolide, diflucortolone valerate, fluprednidene acetate, any pharmaceutically acceptable salt thereof or any combination thereof.

Any of the steroid hormones may be present in the composition in a concentration of up to about 2.5 weight percent, preferably up to about 1 weight percent, more preferably up to about 0.5 weight percent, most preferably up to about 0.1 weight percent.

The antibacterial agent herein includes silver sulfadiazine or nystatine, and the anti-fungal agent includes miconazole. Herein the term "antifungal agent" includes any fungicide and fungistatic agent.

The composition according to the invention may further comprise an additive selected from the group consisting of a skin-softener, a preservative, an emollient, a thickener, a vitamin and a plant extract.

The addition to the composition of a skin softener, preferably glycerol or propylene glycol, provides the composition with properties which add to its suitability for topical use on the skin. The skin softener is present in the composition as obtained in a concentration of 1-5 weight percent, more preferably in a concentration of about 3 weight percent.

In yet another embodiment, the composition further comprises a preservative. Such a preservative can be selected from sorbate or a salt thereof (e.g. potassium sorbate), parabene (methyl, ethyl, propyl and butyl parabens and mixtures), phenoxyethanol, ethylhexylglycerin, iodopropynyl butylcarbamate (IBPC; 3-iodo-2-propynyl-butylcarbamate), a cosmetic chelating agent such as ethylene diamene tetraacetic acid (EDTA) or a salt thereof (e.g., disodium EDTA, tetrasodium EDTA), preferably selected from phenoxyethanol and ethylhexylglycerin, more preferably Euxyl PE 9010. The preservative is used in a concentration of 0.01-5 weight percent of the final product, preferably in a concentration of 0.5-1.5 weight percent of the final product, more preferably in a concentration of 1 weight percent of the final product. The consequence of the addition of a preservative is that the zinc comprising spray-pumpable composition remains stable and usable for a period of at least three years in the original package, stored under ambient temperature and humidity conditions.

In another embodiment, an emollient is comprised by the spray-pumpable composition. Such emollients include triglycerides, preferably coco triglycerides in a concentration of 1-10, more preferably cocoalkyl triglycerides or cetiol LC in a concentration of 5 weight percent. A non-limited list of examples includes propylene glycol dicaprylate/dicaprate, glyceryl caprylate/caprate, pentaerythritol tetracaprylate/tetracaprate or trimethylol propane tricaprylate/tricaprate. Addition of these emollients has the advantage that the skin softening properties of the composition are further improved.

In yet another embodiment, also a thickener is comprised by the spray-pumpable composition. This has the advantage that a more stable composition, or emulsion, is obtained of preferred consistency and viscosity.

Examples of suitable thickeners include fatty acid esters of glycerol in a concentration of 0.1-5 weight percent of the final product, preferably triglycerides and/or diglycerides in a concentration of 0.5-2 weight percent of the final product. Specific, non-limited examples include: Cutina® types, Lamecreme® types, Lanette® types, Lorol® types, Monomuls® types or Stenol® types, more preferably Novata®-types and even more preferably and/or Novata AB in a concentration of 1 weight percent of the final product.

Another aspect of the invention relates to a method for the preparation of a spray-pumpable composition for topical use on the skin comprising the steps of
a) preparing a lipophilic phase comprising at least one active ingredient and optional additives, wherein the at least one active ingredient is zinc oxide or titanium dioxide;
b) preparing an aqueous phase comprising unbound hydrophilic silicon dioxide particles and optional additives, wherein the lipophilic and/or hydrophilic phase comprises an emulsifier, and,
c) mixing the lipophilic phase of step a) with the aqueous phase of step b) to obtain the spray-pumpable composition.

The composition thus obtained is a suspension in an emulsion, more in particular an oil-in water emulsion. The included silicon dioxide is present in the hydrophilic phase of the composition. More in particular, the hydrophilic silicon dioxide particles are in an unbound form and homogenously dispersed in the hydrophilic phase of the composition as obtained. The obtainable emulsion is spray-pumpable and does not require vigorous shaking prior to use. The method according to the invention now allows to prepare such an emulsion, wherein the at least one active ingredient comprises 5-15 weight percent zinc oxide or titanium dioxide.

In case the active ingredient is first dispersed in propylene glycol of up to 5 weight percent of the final composition, it is required to first prepare a dispersion of the active ingredient in propylene glycol before addition thereof to the lipophilic phase. The propylene glycol is used in a concentration of between 1 and 5 weight percent, preferably about 3 weight percent.

Using conventional methods for the preparation of a zinc oxide comprising composition, it was first attempted to add zinc oxide particles having a size distribution of 45 to 90 micron in a variety of manners to existing and conventional emulsions for topical use on the skin. This approach resulted in the provision of emulsions which exhibited significant increased thickening to the extent that such compositions became unsuitable for spray-pumping applications. For example, it was found that the preparation of a composition which comprised more than already 5 weight percent zinc oxide caused such emulsions to become unsuitable for spray-pumping applications.

Subsequently, it was surprisingly found that by using the method of the present invention in combination with a zinc oxide-in-oil formulation, a composition could be obtained which comprises up to about 10 weight percent zinc oxide, which composition displayed a viscosity which still falls within the herein mentioned preferred range. To date, such a method and composition do not appear to be known in the art.

According to a preferred method of the invention, a spray-pumpable composition for topical use on the skin is prepared by the method comprising the steps of
a) preparing a lipophilic phase comprising at least one active ingredient and optional additives, wherein the at least one active ingredient is a zinc-in-oil formulation which preferably comprises zinc oxide particles in a concentration of 40-65 weight percent, preferably 50-65 weight percent, most preferably about 60 weight percent;
b) preparing an aqueous phase comprising unbound hydrophilic silicon dioxide particles and optional additives, wherein the lipophilic and/or hydrophilic phase comprises an emulsifier, and,
c) mixing the lipophilic phase of step a) with the aqueous phase of step b) to obtain the spray-pumpable composition.

This lipophilic phase of step a) may be heated to obtain a sufficiently fluid lipophilic phase which can then be mixed with the aqueous phase of step b).

This method of the invention thus allows the provision of a composition which comprises at least 5, 6, 7, 8, 9 or 10 weight percent zinc oxide. These compositions retain the preferred viscosity as meant herein.

In a more advanced stage of research it was found that it was possible to also obtain a 15 weight percent zinc oxide comprising composition which still exhibited the preferred viscosity as meant herein. Also this composition which comprises up to at least 15 weight percent zinc oxide remained of the preferred viscosity as meant herein. The 15 weight percent zinc oxide comprising composition was prepared by addition of lipophilic zinc oxide particles to the aqueous phase of the present method.

It was surprisingly found that a composition could be obtained having a zinc oxide concentration of more than 10 weight percent zinc oxide in a method for the preparation of a spray-pumpable composition for topical use on the skin, comprising the steps of
a) preparing a lipophilic phase comprising a zinc-in-oil formulation and optional additives;
b) preparing an aqueous phase comprising zinc oxide particles, hydrophilic silicon dioxide and optional additives,
   wherein the lipophilic and/or hydrophilic phase comprises an emulsifier, and,
c) mixing the lipophilic phase of step a) with the aqueous phase of step b) to obtain the spray-pumpable composition.

According to this method, a further amount of lipophilic zinc oxide particles could thus be added to the aqueous medium of step b) while the viscosity of the composition as obtained did not show an increase to the extent that the viscosity of the composition fell outside the herein mentioned range as established using the standardized flow viscosity meter. It has thus been found that it is possible to increase the amount of zinc oxide by at least 5 weight percent of the composition as obtained, through the addition of these lipophilic zinc oxide particles to the aqueous phase. This approach thus allows obtaining a spray-pumpable composition for topical use on the skin which comprises an amount of zinc oxide which exceeds 10 weight percent, in particular 11, 12, 13 14, or up to 15 weight percent or more.

It is also described herein that a spray-pumpable composition can be obtained by adding zinc oxide, or titanium dioxide, particles to the hydrophilic phase in such an amount that a composition is obtained having about 5 weight percent of such particles.

The zinc oxide-in-oil formulation as meant herein is a composition comprising zinc oxide in unsaturated vegetable oil and monounsaturated fatty acids. This in essence anhydrous formulation comprises zinc oxide particles in a concentration of 40-65 weight percent, preferably 50-65 weight percent and most preferably about 60 weight percent zinc oxide particles. The zinc oxide particles comprised by the zinc oxide-in-oil formulation have a size distribution of between 45 and 90 micron.

The amount of monounsaturated fatty acids of the zinc oxide-in-oil formulation is about up to 1 weight percent. The amount of unsaturated vegetable oil comprises the remaining portion of the zinc oxide-in-oil formulation, i.e. 30-50 weight percent, more preferably 35-45 weight percent, most preferably about 40 weight percent.

The zinc oxide-in-oil formulation is preferably homogenized before the addition to the lipophilic phase as this ensures the addition of a uniform amount of zinc oxide and facilitates later processing steps, such as mixing or homogenization steps. The use of the zinc-in-oil formulation may require heating of the lipophilic phase in order to obtain a fluid lipophilic phase which would facilitate mixing with the aqueous phase. It lies well within the capabilities of the skilled person to determine whether heating would be necessary during the preparation of the composition as claimed.

It is further described herein that the zinc oxide particles are added to the aqueous medium of step b) while the emulsifier of step a) is a C₁₄-C₂₂ alcohols and/or C₁₂-C₂₀ glucosides emulsifier. Using such an emulsifier, it was found that a spray-pumpable composition could be obtained which comprised a total of at least 15 or more weight percent zinc oxide particles. Using the standardized flow viscosity meter, the viscosity of the 15 weight percent or more zinc oxide composition according to this embodiment was found to fall within the preferred ranged and to be suitable for topical use on the skin.

Using an emollient according to the disclosure, such as triglycerides, preferably coco triglycerides, more preferably cocoalkyl triglycerides or cetiol LC, it is possible to obtain a homogenous lipophilic phase in step a) of the method according to the invention, without the need of using a homogenizer of colloid mill. The use of the emollients according to the disclosure reduces the need to use more physically demanding means of homogenizing.

It is further contemplated that a composition according to the present invention can be provided wherein zinc oxide particles may be exchanged for titanium dioxide particles. Such a composition may comprise from anywhere between 5 to 15 weight percent titanium dioxide, such as 6, 7, 8, 9, 10, 11, 12, 13, or 14 weight percent. Such compositions may find use as sun cream, sun blocker or the like.

Another aspect of the invention relates to the composition of the invention for use in the treatment of a topical skin disorder, in a mammal, in particular a human being, wherein the topical skin disorder is preferably a rash, diaper rash in particular, sun burn, eczema, an inflammation or an infection. Using the composition which comprises 5, 10 or 15 weight percent zinc oxide or titanium dioxide, it is possible to treat skin disorders in an animal, such as a mammal, or a human being.

Using the spray-pumpable composition according to the invention, it is possible to apply the zinc oxide, or titanium dioxide or other active ingredients in high amounts by one or few applications or dosages such that sufficient amounts of the active ingredient will be applied onto the skin by the single or few applications.

The composition comprising zinc oxide can for example be used in the treatment of a rash, diaper rash in particular, sun burn, eczema, an inflammation, an infection, or other skin disorders.

The composition further comprising a steroid hormone can for example be used in the treatment of psoriasis, rash, eczema, an inflammation, an infection, or other skin disorders.

Yet another aspect of the present invention relates to the use of unbound hydrophilic silicon dioxide particles, preferably colloidal hydrophilic silicon dioxide particles, in a method for the preparation of aspray-pumpable suspension in an emulsion, preferably a suspension in an oil-in-water emulsion, wherein the spray-pumpable suspension in an emulsion comprises 5-15 weight percent zinc oxide or titanium dioxide.

### FIGURES

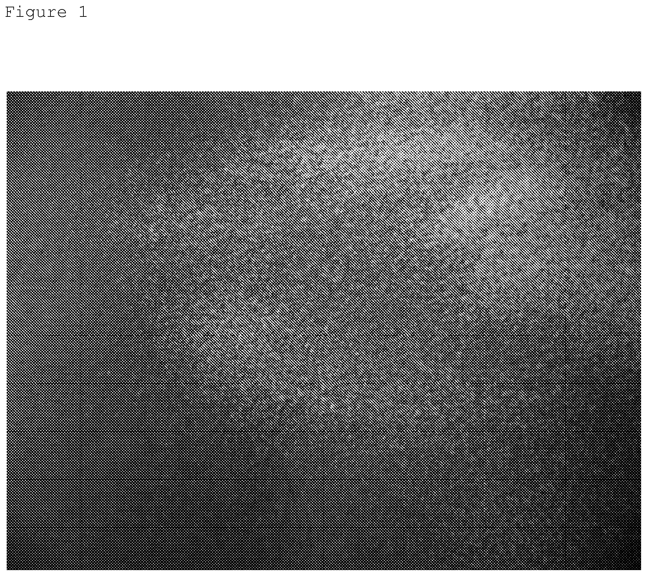

Figure 1 shows a representative spray-pattern of a spray-pumpable composition according to the invention.

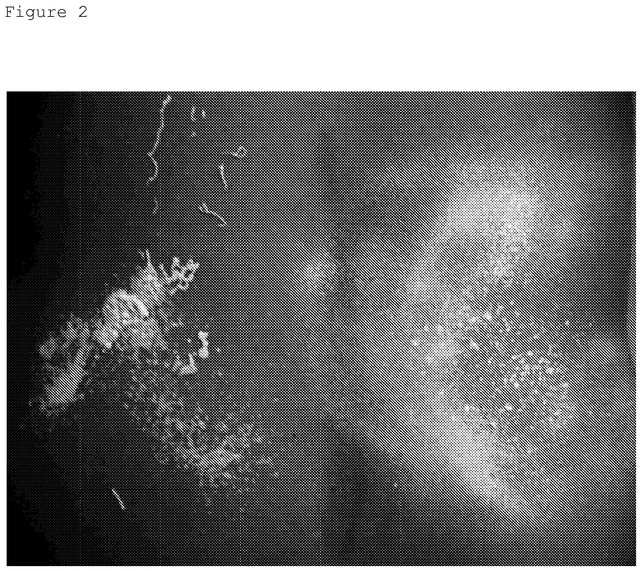

Figure 2 shows two spray-patterns obtained after application on human skin using the Emsar fine-mist spray system. The left-hand-sided pattern is obtained from application of a non-spray-pumpable composition as demonstrated by the formation of thick blobs and long strands of the applied composition. The right-hand-sided part of the picture displays a representative pattern obtained from the application of the 10 weight percent zinc oxide spray-pumpable composition according to the invention.

To attain an evenly-spread, thin layer, the left-hand-sided applied composition requires longer rubbing after topical skin application than the right-hand-sided applied composition. The right-hand-sided applied composition can be evenly spread over human skin with a few strokes while also the amount of residual composition left on the fingers is less compared to the left-applied composition.

### EXAMPLES

### Example 1. Preparation of 10 weight percent zinc oxide milk spray for topical use on the skin.

A spray-pumpable zinc oxide composition of a total of 100 gram was prepared according to the below method.

A lipophilic phase was prepared by mixing in a first holder of 3 gram of an emulsifier, such as Montanov L. or Montanov 68, with 5 gram of an emollient (e.g Cetiol LC) and 1 gram of a hard fat (e.g Novata AB), and 16.6 gram of a zinc oxide-in-oil formulation comprising 60 weight percent zinc oxide particles, 39.3 weight percent monounsaturated vegetable oil and 0.7 weight percent fatty acids, and heating the lipophilic phase to about 75ºC under moderate stirring.

A hydrophilic phase was prepared by mixing in a second holder of 3 gram 100% glycerol, 1 gram of a preservative (e.g. Euxyl PE 9010), 0.1 gram hydrophilic silicium dioxide (e.g. Aerosil 200) and up to 70.2 gram of water, and heating of this phase to about 80ºC under moderate stirring to obtain a fluid hydrophilic phase.

Subsequently, the hydrophilic phase is added to the lipophilic phase under continuous, moderate stirring. The mixture is allowed to cool under continuous stirring after which any remaining water of the 70.2 gram thereof is added to obtain the 10 weight percent zinc oxide comprising composition.

This method of preparing the spray-pumpable composition by mixing has the advantage that physically more demanding methods for preparation of the end-product are not necessary anymore. According to conventional methodology, it is necessary to use a colloid mill or homogenizer to obtain a homogenous emulsion.

Composition of 100 gram 10 weight percent milk spray.

| | |
|---|---|
| Emulsifier (e.g. Montanov L./Montanov 68) | 3 g |
| Emollient (e.g. Cetiol LC) | 5 g |
| Thickener (e.g.Novata AB) | 1 g |
| Zinc oxide-in-oil formulation | 16.6 g |
| Glycerol (100 %) | 3 g |
| Conservative (Euxyl PE 9010) | 1 g |
| Hydrophilic silicium dioxide | 0.1 g |

An amount of water to obtain 100 g of the composition

### Example 2. Preparation of 15 weight percent zinc oxide milk spray for topical use on the skin.

In a second series of experiments, the amount of zinc oxide in the composition was attempted to be raised even further.

To meet this goal, a zinc oxide comprising composition was prepared according to the method of example 1, with the exception that 5 gram lipophilic zinc oxide particles were added to the hydrophilic phase and 65.2 grams water.

Composition of 100 gram 15 weight percent milk spray.

| | |
|---|---|
| Emulsifier (e.g. Montanov L.) | 3 g |
| Emollient (e.g. Cetiol LC) | 5 g |
| Thickener (e.g. Novata AB) | 1 g |
| Zinc oxide-in oil formulation | 16.6 g |
| Glycerol | 3 g |
| Conservative (Euxyl PE 9010) | 1 g |
| Hydrophilic silicium dioxide | 0.1 g |
| Zinc oxide (90) particles | 5 g |

An amount of water to obtain 100 g of the composition

### Example 3. Preparation of a triamcinolon acetonide derma-spray.

A lipophilic phase was prepared by mixing in a first holder of 3 gram of an emulsifier (e.g. Montanov L.), with 5 gram of an emollient (e.g Cetiol LC) and 1 gram of a hard fat (e.g Novata AB) to which 3.1 gram of a dispersion of 0.1 gram triamcinolon acetonide in 3 gram propylene glycol is added. The lipophilic phase is subsequently heated to about 75ºC under moderate stirring.

A hydrophilic phase was prepared by mixing in a second holder of 1 gram preservative (e.g. Euxyl PE 9010), 0.1 gram hydrophilic silicon dioxide (e.g. Aerosil 200) with up to 86.9 gram of water, and heating of this phase to about 80ºC under moderate stirring.

Subsequently, the hydrophilic phase is added to the lipophilic phase under continuous, moderate stirring. Homogenizing is performed using a pre-warmed turrax mixer for 20 seconds at 3000 rpm. The mixture is allowed to cool under continuous stirring after which any remaining water of the 86.9 gram water is added to obtain a 0.1 weight percent triamcinolon acetonide derma-spray.

### Composition of 100 gram triamcinolon derma-spray.

| | |
|---|---|
| Emulsifier (e.g. Montanov 68) | 3 g |
| Emollient (e.g. Cetiol LC) | 5 g |
| Thickener (e.g. Novata AB PH) | 1 g |
| Triamcinolon acetonide | 0.1 g |
| Propyleen glycol | 3 g |
| Preservative (e.g. Euxyl PE 9010) | 1 g |
| Hydrophilic silicium dioxide | 0.1 g |

An amount of water to obtain 100 g derma-spray

## Claims

1. Spray-pumpable composition for topical use comprising unbound hydrophilic silicon dioxide particles, an emulsifier, at least one active ingredient, and optionally one or more additives, wherein the at least one active ingredient comprises 5-15 weight percent zinc oxide or titanium dioxide.

2. Composition according to claim 1, wherein the hydrophilic silicon dioxide has a specific surface area of between 130 and 380 m²/g, preferably between 150 and 300 m²/g, more preferably between 175 and 225 m²/g, most preferably around 200 m²/g.

3. Composition according to claim 1 or 2, wherein the composition is a suspension of hydrophilic silicon dioxide in an emulsion, in particular a suspension of hydrophilic silicon dioxide in an oil-in-water emulsion, and wherein the hydrophilic silicon dioxide is preferably suspended in the water phase of an emulsion, in particular an oil-in-water emulsion.

4. Composition according to any of the claims 1-3, wherein the hydrophilic silicon dioxide is present in a concentration of 0.01-0.5 weight percent, preferably in a concentration of about 0.1 weight percent.

5. Composition according to any of the claims 1-4, wherein the zinc oxide or titanium dioxide is present as particles having a size distribution in the range of 45-90 micron.

6. Composition according to any of the claims 1-5, wherein the emulsifier is a hydrophilic or lipophilic non-ionic surfactant, preferably a lipophilic non-ionic surfactant, more preferably a gluco-lipid emulsifier or a gluco-lipid co-emulsifier and wherein the emulsifier preferably comprises cetearylalcohols and/or cetearylglucosides or C₁₄-C₂₂ alcohols and/or C₁₂-C₂₀ glucosides or a mixture thereof and is preferably present in a concentration of 0.1-10 weight percent, preferably 0.5-5 weight percent, more preferably 1-4 weight percent and most preferably about 3 weight percent.

7. Composition according to any of the claims 1-6, wherein the additive is selected from the group consisting of
- a skin-softener, preferably selected from the group consisting of glycerol and propylene glycol, preferably in a concentration of 1-5 weight percent, more preferably in a concentration of about 3 weight percent;
- a preservative, preferably selected from the group consisting of sorbate or a salt thereof; a parabene such as methylparabene, ethylparabene, propylparabene or butylparabene or a mixture thereof; phenoxyethanol; ethylhexylglycerin; iodopropynyl butylcarbamate; and a cosmetic chelating agent, such as ethylene diamene tetraacetic acid (EDTA) or a salt thereof; preferably selected from phenoxyethanol and ethylhexylglycerin, wherein more preferably the preservative is Euxyl PE 9010, and preferably used in a concentration of 0.01-5 weight percent, preferably in a concentration of 0.5-1.5 weight percent, more preferably in a concentration of 1 weight percent;
- an emollient, preferably selected from the group consisting of triglycerides, preferably coco triglycerides, more preferably cocoalkyl triglycerides or cetiol LC, and preferably present in a concentration of 1-10 weight percent, preferably in a concentration of about 5 weight percent;
- a thickener, preferably selected from the group consisting of fatty acid esters of glycerol, preferably triglycerides and/or diglycerides, and preferably present in a concentration of 0.1-5 weight percent, preferably in a concentration of 0.5-2 weight percent, more preferably about 1 weight percent;
- a vitamin and
- a plant extract.

8. Method for the preparation of a spray-pumpable composition for topical use, comprising the steps of
a) preparing a lipophilic phase comprising at least one active ingredient and optional additives, wherein the at least one active ingredient is zinc oxide or titanium dioxide;
b) preparing an aqueous phase comprising unbound hydrophilic silicon dioxide particles and optional additives, wherein the lipophilic and/or hydrophilic phase comprises an emulsifier, and,
c) mixing the lipophilic phase of step a) with the aqueous phase of step b) to obtain the spray-pumpable composition.

9. Method according to claim 8, wherein the composition as obtained is a suspension in an emulsion, in particular a suspension in an oil-in-water emulsion.

10. Method according to claim 8 or 9, wherein the hydrophilic silicon dioxide has a specific surface area of between 130 and 380 m²/g, preferably between 150 and 300 m²/g, more preferably between 175 and 225 m²/g, most preferably around 200 m²/g.

11. Method according to any of the claims 8-10, wherein the zinc oxide of step a) is added as a zinc oxide-in-oil formulation, which preferably comprises zinc oxide particles in a concentration of 40-65 weight percent, preferably 50-65 weight percent, most preferably about 60 weight percent.

12. Method according to claim 11, wherein zinc oxide particles are added to the aqueous phase of step b) in such an amount that the composition as obtained comprises an additional 5 weight percent zinc oxide.

13. Composition according to any of the claims 1-7 or obtainable by the method of any of the claims 8-12 for use in the treatment of a topical skin disorder in a mammal, in particular a human being, wherein the topical skin disorder is preferably a rash, diaper rash in particular, sun burn, eczema, an inflammation or an infection.

14. Use of unbound hydrophilic silicon dioxide particles in a method for the preparation of a spray-pumpable suspension in an emulsion, preferably a suspension in an oil-in-water emulsion, wherein the spray-pumpable suspension in an emulsion comprises 5-15 weight percent zinc oxide or titanium dioxide.

## Patentansprüche

1. Sprühpumpzusammensetzung für die topische Verwendung, die aufweist ungebundene hydrophile Siliziumdioxid-Teilchen, einen Emulgator, wenigstens einen aktiven Inhaltsstoff und optional einen oder mehrere Zusatzstoffe, wobei der wenigstens eine aktive Inhaltsstoff 5 - 15 Gewichtsprozent Zinkoxid oder Titandioxid aufweist.

2. Zusammensetzung nach Anspruch 1, wobei das hydrophile Siliziumdioxid einen spezifischen Oberflächenbereich von zwischen 130 und 380 m²/g hat, vorzugsweise zwischen 150 und 300 m²/g, bevorzugter zwischen 175 und 225 m²/g, besonders bevorzugt um 200 m²/g.

3. Zusammensetzung nach Anspruch 1 oder 2, wobei die Zusammensetzung eine Suspension aus hydrophilem Siliziumdioxid in einer Emulsion ist, im Besonderen eine Suspension von hydrophilem Siliziumdioxid in einer Öl-in-Wasser-Emulsion, und wobei das hydrophile Siliziumdioxid vorzugsweise in der Wasserphase einer Emulsion suspendiert ist, im Besonderen einer Öl-in-Wasser-Emulsion.

4. Zusammensetzung nach einem der Ansprüche 1 bis 3, wobei das hydrophile Siliziumdioxid in einer Konzentration von 0,01 - 0,5 Gewichtsprozent, vorzugsweise in einer Konzentration von etwa 0,1 Gewichtsprozent vorhanden ist.

5. Zusammensetzung nach einem der Ansprüche 1 bis 4, wobei das Zinkoxid oder Titandioxid als Teilchen vorliegt, die eine Größenverteilung im Bereich von 45 - 90 Mikrometer haben.

6. Zusammensetzung nach einem der Ansprüche 1 bis 5, wobei der Emulgator ein hydrophiler oder lipophiler nicht-ionischer oberflächenaktiver Stoff ist, bevorzugt ein lipophiler nicht-ionischer oberflächenaktiver Stoff, bevorzugter ein glukolipider Emulgator oder ein glukolipider Co-Emulgator, und wobei der Emulgator vorzugsweise Cetearylalkohole und/oder Cetearylglukoside oder C₁₄-C₂₂-Alkohole und/oder C₁₂-C₂₀-Glukoside oder eine Mischung davon aufweist und bevorzugt in einer Konzentration von 0,1 - 10 Gewichtsprozent, vorzugsweise 0,5 - 5 Gewichtsprozent, bevorzugter 1-4 Gewichtsprozent und besonders bevorzugt etwa 3 Gewichtsprozent vorhanden ist.

7. Zusammensetzung nach einem der Ansprüche 1 bis 6, wobei der Zusatzstoff ausgewählt ist aus der Gruppe bestehend aus
- einem Hautweichmacher, der vorzugsweise ausgewählt ist aus der Gruppe bestehend aus Glycerin und Propylenglykol, vorzugsweise in einer Konzentration von 1-5 Gewichtsprozent, bevorzugter in einer Konzentration von etwa 3 Gewichtsprozent;
- einem Konservierungsstoff, der vorzugsweise ausgewählt ist aus der Gruppe bestehend aus Sorbat oder einem Salz davon; einem Paraben, wie Methylparaben, Ethylparaben, Propylparaben oder Butylparaben oder einer Mischung davon; Phenoxyethanol; Ethylhexylglycerin; Iodopropynylbutylcarbamat; und einem kosmetischen Chelatbildner, wie Ethylendiamentetraessigsäure (EDTA) oder einem Salz davon; vorzugsweise ausgewählt aus Phenoxyethanol und Ethylhexylglycerin, wobei das Konservierungsmittel bevorzugter Euxyl PE 9010 ist, und vorzugsweise in einer Konzentration von 0,01 - 5 Gewichtsprozent, bevorzugt in einer Konzentration von 0,5 - 1,5 Gewichtsprozent, bevorzugter in einer Konzentration von 1 Gewichtsprozent verwendet wird;
- einem Weichmacher, der vorzugsweise ausgewählt ist aus der Gruppe bestehend aus Triglyceriden, vorzugsweise Coco-Triglyceriden, bevorzugter Cocoalkyl-Triglyceriden oder Cetiol LC, und vorzugsweise in einer Konzentration von 1-10 Gewichtsprozent, vorzugsweise in einer Konzentration von etwa 5 Gewichtsprozent vorhanden ist;
- einem Verdickungsmittel, das vorzugsweise ausgewählt ist aus der Gruppe bestehend aus Fettsäureestern von Glycerin, vorzugsweise Triglyceride und/oder Diglyceride, und vorzugsweise in einer Konzentration von 0,1 - 5 Gewichtsprozent vorhanden ist, vorzugsweise in einer Konzentration von 0,5 - 2 Gewichtsprozent, bevorzugter etwa 1 Gewichtsprozent;
- einem Vitamin und
- einem Pflanzenextrakt.

8. Verfahren zum Herstellen einer Sprühpumpzusammensetzung zur topischen Verwendung, das die Schritte aufweist
a) Herstellen einer lipophilen Phase, die wenigstens einen aktiven Inhaltsstoff und optionale Zusatzstoffe aufweist, wobei der wenigstens eine aktive Inhaltsstoff Zinkoxid oder Titandioxid ist;
b) Herstellen einer wässrigen Phase, die ungebundene hydrophile Siliziumdioxid-Teilchen und optionale Zusatzstoffe aufweist, wobei die lipophile und/oder hydrophile Phase einen Emulgator aufweist, und
c) Mischen der lipophilen Phase von Schritt a) mit der wässrigen Phase von Schritt b), um eine Sprühpumpzusammensetzung zu erhalten.

9. Verfahren nach Anspruch 8, wobei die erhaltene Zusammensetzung eine Suspension in einer Emulsion ist, im Besonderen eine Suspension in einer Öl-in-Wasser-Emulsion.

10. Verfahren nach Anspruch 8 oder 9, wobei das hydrophile Siliziumdioxid einen spezifischen Oberflächenbereich von zwischen 130 und 380 m²/g hat, vorzugsweise zwischen 150 und 300 m²/g, bevorzugter zwischen 175 und 225 m²/g, besonders bevorzugt um 200 m²/g.

11. Verfahren nach einem der Ansprüche 8 bis 10, wobei das Zinkoxid von Schritt a) als eine Zinkoxid-in-Öl-Formel hinzugefügt wird, die vorzugsweise Zinkoxid-Teilchen in einer Konzentration von 40 - 65 Gewichtsprozent, bevorzugt 50 - 65 Gewichtsprozent, besonders bevorzugt um 60 Gewichtsprozent aufweist.

12. Verfahren nach Anspruch 11, wobei die Zinkoxiden-Teilchen zu der wässrigen Phase von Schritt b) in einer solchen Menge hinzugefügt werden, dass die erhaltene Zusammensetzung weitere 5 Gewichtsprozent Zinkoxid aufweist.

13. Zusammensetzung nach einem der Ansprüche 1 bis 7 oder durch das Verfahren nach einem der Ansprüche 8 bis 12 erhaltbar zur Verwendung in der Behandlung einer topischen Hauterkrankung in einem Säugetier, in Besonderen einem Menschen, wobei die oberflächliche Hauterkrankung vorzugsweise ein Hautausschlag, im Besonderen ein Windelausschlag, ein Sonnenbrand, ein Ekzem, eine Entzündung oder eine Infektion ist.

14. Verwendung von ungebundenen hydrophilen Siliziumdioxid-Teilchen in einem Verfahren zum Herstellen einer Sprühpumpsuspension in einer Emulsion, vorzugsweise einer Suspension in einer Öl-in-Wasser-Emulsion, wobei die Sprühpumpsuspension in einer Emulsion 5 - 15 Gewichtsprozent Zinkoxid oder Titandioxid aufweist.

## Revendications

1. Composition pompable pour atomiseur pour utilisation topique comprenant des particules de dioxyde de silicium hydrophiles non liées, un émulsifiant, au moins un principe actif et facultativement un ou plusieurs additifs, dans laquelle l'au moins un principe actif comprend de 5 à 15 pour cent en poids d'oxyde de zinc ou de dioxyde de titane.

2. Composition selon la revendication 1, dans laquelle le dioxyde de silicium hydrophile présente une superficie spécifique entre 130 et 380 m²/g, de préférence entre 150 et 300 m²/g, plus préférablement entre 175 et 225 m²/g, idéalement d'environ 200 m²/g.

3. Composition selon la revendication 1 ou 2, dans laquelle la composition est une suspension de dioxyde de silicium hydrophile dans une émulsion, en particulier une suspension de dioxyde de silicium hydrophile dans une émulsion huile dans eau, et dans laquelle le dioxyde de silicium hydrophile est de préférence suspendu dans la phase eau d'une émulsion, en particulier une émulsion huile dans eau.

4. Composition selon l'une quelconque des revendications 1 à 3, dans laquelle le dioxyde de silicium hydrophile est présent en une concentration de 0,01 à 0,5 pour cent en poids, de préférence en une concentration d'environ 0,1 pour cent en poids.

5. Composition selon l'une quelconque des revendications 1 à 4, dans laquelle l'oxyde de zinc ou le dioxyde de titane est présent sous forme de particules présentant une distribution granulométrique dans la plage de 45 à 90 micromètres.

6. Composition selon l'une quelconque des revendications 1 à 5, dans laquelle l'émulsifiant est un tensioactif non ionique hydrophile ou lipophile, de préférence un tensioactif non ionique lipophile, plus préférablement un émulsifiant gluco-lipide ou un co-émulsifiant gluco-lipide et dans laquelle l'émulsifiant comprend de préférence des cétéarylalcools et/ou des cétéarylglucosides ou des alcools C₁₄-C₂₂ et/ou des glucosides C₁₂-C₂₀ ou un mélange de ceux-ci et est de préférence présent en une concentration de 0,1 à 10 pour cent en poids, de préférence de 0,5 à 5 pour cent en poids, plus préférablement de 1 à 4 pour cent en poids et idéalement d'environ 3 pour cent en poids.

7. Composition selon l'une quelconque des revendications 1 à 6, dans laquelle l'additif est sélectionné à partir du groupe constitué de
- un adoucissant pour la peau, de préférence sélectionné à partir du groupe constitué de glycérol et de propylène glycol, de préférence en une concentration de 1 à 5 pour cent en poids, plus préférablement en une concentration d'environ 3 pour cent en poids ;
- un conservateur, de préférence sélectionné à partir du groupe constitué de sorbate ou d'un sel de celui-ci ; un parabène tel que du méthylparabène, de l'éhtylparabène, du propylparabène ou du butylparabène ou un mélange de ceux-ci ; du phénoxyéthanol ; de l'éthylhexylglycérine ; de l'iodopropynyle butylcarbamate ; et un agent de chélation cosmétique, tel que l'acide éthylène diamine tétraacétique (EDTA) ou un sel de celui-ci ; de préférence sélectionné à partir de phénoxyéthanol et d'éthylhexylglycérine, dans laquelle plus préférablement le conservateur est de l'Euxyl PE 9010, et de préférence utilisé en une concentration de 0,01 à 5 pour cent en poids, de préférence en une concentration de 0,5 à 1,5 pour cent en poids, plus préférablement en une concentration de 1 pour cent en poids ;
- un émollient, de préférence sélectionné à partir du groupe constitué de triglycérides, de préférence de coco triglycérides, plus préférablement de cocoalkyl triglycérides ou cétiol LC, et de préférence présent en une concentration de 1 à 10 pour cent en poids, de préférence en une concentration d'environ 5 pour cent en poids ;
- un épaississant, de préférence sélectionné à partir du groupe constitué d'esters d'acide gras de glycérol, de préférence de triglycérides et/ou de diglycérides, et de préférence présent en une concentration de 0,1 à 5 pour cent en poids, de préférence en une concentration de 0,5 à 2 pour cent en poids, plus préférablement d'environ 1 pour cent en poids ;
- une vitamine et
- un extrait de plante.

8. Procédé pour la préparation d'une composition pompable pour atomiseur pour utilisation topique, comprenant les étapes de
a) préparation d'une phase lipophile comprenant au moins un principe actif et des additifs optionnels, dans lequel l' au moins un principe actif est l'oxyde de zinc ou le dioxyde de titane ;
b) préparation d'une phase aqueuse comprenant des particules de dioxyde de silicium hydrophile non liées et des additifs optionnels, dans lequel la phase lipophile et/ou la phase hydrophile comprennent un émulsifiant, et,
c) mélange de la phase lipophile de l'étape a) avec la phase aqueuse de l'étape b) pour obtenir la composition pompable pour atomiseur.

9. Procédé selon la revendication 8, dans lequel la composition telle qu'obtenue est une suspension dans une émulsion, en particulier une suspension dans une émulsion huile dans eau.

10. Procédé selon la revendication 8 ou 9, dans lequel le dioxyde de silicium hydrophile présente une superficie spécifique entre 130 et 380 m²/g, de préférence entre 150 et 300 m²/g, plus préférablement entre 175 et 225 m²/g, idéalement d'environ 200 m²/g.

11. Procédé selon l'une quelconque des revendications 8 à 10, dans lequel l'oxyde de zinc de l'étape a) est ajouté en tant que formulation d'oxyde de zinc dans huile, qui comprend de préférence des particules d'oxyde de zinc en une concentration de 40 à 65 pour cent en poids, de préférence de 50 à 65 pour cent en poids, idéalement d'environ 60 pour cent en poids.

12. Procédé selon la revendication 11, dans lequel les particules d'oxyde de zinc sont ajoutées à la phase aqueuse de l'étape b) en une quantité telle que la composition telle qu'obtenue comprend 5 pour cent en poids supplémentaires d'oxyde de zinc.

13. Composition selon l'une quelconque des revendications 1 à 7 ou pouvant être obtenue par le procédé selon l'une quelconque des revendications 8 à 12 pour utilisation dans le traitement d'un trouble topique de la peau chez un mammifère, en particulier un être humain, chez lequel le trouble topique de la peau est de préférence une éruption cutanée, un érythème fessier en particulier, un coup de soleil, de l'eczéma, une inflammation ou une infection.

14. Utilisation de particules de dioxyde de silicium hydrophile non liées dans un procédé pour la préparation d'une suspension pompable pour atomiseur dans une émulsion, de préférence une suspension dans une émulsion huile dans eau, dans laquelle la suspension pompable pour atomiseur dans une émulsion comprend de 5 à 15 pour cent en poids d'oxyde de zinc ou de dioxyde de titane.
